Europäisches Patentamt

**European Patent Office**

Office européen des brevets

(11) Numéro de publication: **0 272 295 B1**

# FASCICULE DE BREVET EUROPEEN

(12)

(45) Date de publication de fascicule du brevet: **02.09.92**  (51) Int. Cl.⁵: **C12N 9/52**, A23K 1/165, C12R 1/54

(21) Numéro de dépôt: **87904053.3**

(22) Date de dépôt: **19.06.87**

(86) Numéro de dépôt internationale :
**PCT/FR87/00233**

(87) Numéro de publication internationale :
**WO 87/07905 (30.12.87 87/29)**

---

(54) **PROCEDE DE PRODUCTION D'UN NOUVEAU COMPLEXE PROTEOLYTIOUE STIMULANT L'ACTIVITE DU PANCREAS ET SES APPLICATIONS EN ZOOTECHNIE.**

---

(30) Priorité: **20.06.86 FR 8608940**

(43) Date de publication de la demande:
**29.06.88 Bulletin 88/26**

(45) Mention de la délivrance du brevet:
**02.09.92 Bulletin 92/36**

(84) Etats contractants désignés:
**AT BE CH DE FR GB IT LI LU NL SE**

(56) Documents cités:
**FR-A- 2 034 559**
**GB-A- 1 133 579**

**Biotechnology and Bioengineering, vol. XXIII, No 1, January 1981, John Wiley & Sons, Inc., (New York, US), Toshinori Kokubu et al.: "Protease production by immobilized mycelia of Streptomyces fradiae", pages 23-39 See page 29, "Summary" and page 38: "Discussion"**

(73) Titulaire: **HOOREMAN, Dominique**
**5, rue de Lisbonne**
**F-75008 Paris(FR)**

Titulaire: **HOOREMAN, Denis**
**4, rue de la Vallée**
**F-60800 Crépy en Valois(FR)**

Titulaire: **HOOREMAN, Jean-Noel**
**23, rue de Sablonville**
**F-92200 Neuilly sur Seine(FR)**

Titulaire: **HOOREMAN, Hervé**
**17bis, avenue ch. de Gaulle**
**F-95160 Montmorency(FR)**

Titulaire: **HOOREMAN, Michel**
**10, rue du Delta**
**F-75009 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

(72) Inventeur: **HOOREMAN, Dominique**
**5, rue de Lisbonne**
**F-75008 Paris(FR)**
Inventeur: **HOOREMAN, Denis**
**4, rue de la Vallée**
**F-60800 Crépy en Valois(FR)**
Inventeur: **HOOREMAN, Jean-Noel**
**23, rue de Sablonville**
**F-92200 Neuilly sur Seine(FR)**
Inventeur: **HOOREMAN, Hervé**
**17bis, avenue ch. de Gaulle**
**F-95160 Montmorency(FR)**
Inventeur: **HOOREMAN, Michel**
**10, rue du Delta**
**F-75009 Paris(FR)**

(74) Mandataire: **Burtin, Jean-François**
**Cabinet GEFIB, 59, rue Edouard-Vaillant**
**F-92300 Levallois-Perret(FR)**

## Description

Dans son brevet français n° 2 034 559, le demandeur a décrit un procédé de production de complexes protéolytiques à partir de Streptomyces fradiae, et les emplois possibles de ces complexes notamment en zootechnie.

L'expérience a montré que les résultats donnés par la mise en application de ce brevet étaient en général positifs, mais irréguliers ou insuffisants, de sorte que les emplois de ces complexes sont restés très limités.

La présente demande a pour objet de décrire un procédé qui permet d'obtenir un complexe nouveau, particulier, ayant une composition plus précisément fixée, et de définir les conditions qui permettent à ce nouveau complexe de donner avec régularité des améliorations importantes en zootechnie.

La présente demande concerne aussi le nouveau complexe protéolytique obtenu par application de ce procédé.

Dans son brevet anglais n° 1 133 579, la société SHIONOGI a indiqué que Streptomyces fradiae pouvait produire au moins 5 protéases, désignées par $I_a$, $I_b$, II, III, IV, et 2 peptidases. Le demandeur a également obtenu à partir de Streptomyces fradiae des enzymes qui, ayant pratiquement les mêmes propriétés générales, sont du même type que celles qui sont décrites dans le brevet SHIONOGI. Dans la présente demande, par souci de clarté, on a adopté pour désigner ces enzymes les mêmes chiffres romains que dans ledit brevet.

Le demandeur a constaté que les complexes composés exclusivement des protéases du type II, III, IV provoquent la réduction modérée de la viscosité du mucus intestinal prescrite dans son brevet initial et donnent en général des résultats positifs en zootechnie, mais irréguliers ou insuffisants; parmi tous ces complexes, seul le complexe particulier, caractérisé par une plus grande abondance de la protéase du type II par rapport aux protéases du type III ou IV, peut donner avec régularité une amélioration importante des performances zootechniques.

Le demandeur a également constaté que les complexes globaux, composés de 5 types de protéases et des 2 types de peptidases, provoquent une réduction excessive de la viscosité du mucus intestinal, et sont agressifs pour la muqueuse; ils sont donc inutilisables en zootechnie.

C'est pourquoi on a cherché à obtenir le complexe particulier composé exclusivement des protéases du type II, III, IV, avec prépondérance de la protéase du type II. C'est ce nouveau complexe qui fait l'objet des revendications 5 à 7 de la présente demande.

## PROCEDE DE PRODUCTION

Le complexe selon l'invention est obtenu dans les conditions suivantes:

### Fermentation

On utilise une souche de Streptomyces fradiae, par exemple la souche typique WAKSMAN 3535, déposée à l'American Type Culture Collection (en abrégé ATCC) sous le n° 10.745. Cette souche peut être périodiquement régénérée par les techniques classiques de dissociation sur milieu gélosé pour éliminer d'éventuels mutants spontanés à faible activité protéolytique. Cette souche peut être aussi améliorée par les techniques classiques de mutation provoquée en vue d'obtenir une augmentation de l'activité protéolytique.

On prépare des précultures de cette souche, d'abord en fioles agitées, puis dans un fermenteur intermédiaire qu'on inocule ensuite dans le fermenteur de production industrielle. Le milieu de production peut avoir la composition suivante: farine de soja: 15 g/l; glucose: 30 g/l; phosphate bipotassique: 1 g/l; carbonate de calcium: 10 g/l; après stérilisation, son pH est voisin de 7,0; on fixe la température de fermentation à 28°C, et l'aération à 0,3 volume d'air stérile par volume de milieu et par minute.

Le pH du milieu, initialement voisin de 7,0, commence par baisser et atteint 6,5 après environ 10 heures de fermentation; il remonte ensuite rapidement et atteint 7,5 après environ 20 heures de fermentation, puis il continue à remonter plus lentement pour atteindre finalement 8,2.

On a constaté qu'il était très important de maintenir ce pH entre 7,5 et 7,0 par addition automatique d'une solution acide, par exemple de l'acide chlorhydrique 3N, ce qui permet:
- d'éviter la fragmentation du mycelium, et la libération dans le milieu de certains métabolites secondaires, des protéases du type $I_a$, $I_b$, et des peptidases; ces métabolites secondaires et ces enzymes demeurent ainsi endocellulaires;
- d'augmenter la production des protéases du type II, III, IV qui sont des enzymes principalement exocellulaires;
- d'obtenir une augmentation spécialement importante pour la production de la protéase du type II.

La durée de fermentation peut varier de 72 à 96 heures, et le titre final du milieu est d'environ 15 000 Unités Anson par ml; il dépasse donc de beaucoup le titre de 3 000 Unités Anson par ml indiqué dans le brevet français n° 2 034 559.

Une unité Anson (en abrégé U.A.) est ici définie comme étant la quantité d'enzyme qui, incubée pendant 10 minutes à 25°C et à pH 7,5 en présence d'hémoglobine dénaturée, libère de ce substrat l'équivalent de 1 µg de tyrosine, déterminée par absorption spectrophotométrique à 280 nm sur le filtrat non précipitable à l'acide trichloracétique.

Extraction

Le mycelium est séparé du milieu de production par une filtration clarifiante.

Le complexe, au lieu d'être extrait par les techniques classiques de séparation des enzymes (précipitations successives, passages sur colonne), est extrait par une technique plus récente, l'ultrafiltration.

On a recherché quelles étaient les membranes d'ultrafiltration pouvant conduire aux meilleurs rendements; les membranes qui ont un seuil de coupure correspondant à un poids moléculaire de 30 000 ne conviennent pas parce qu'elles laissent passer plus de 50% du complexe; les membranes qui ont un seuil de coupure correspondant à un poids moléculaire de 1 000 ne conviennent pas parce qu'elles ne permettent pas de séparer le complexe de certains métabolites secondaires à faible poids moléculaire On a finalement constaté qu'il convenait d'utiliser des membranes qui ont un seuil de coupure correspondant à un poids moléculaire compris entre 2 000 et 12 000; on peut par exemple utiliser la membrane de polysulfone vendue par la Société MILLIPORE sous la dénomination PTGC, qui a un seuil de coupure correspondant à un poids moléculaire de 10 000.

Le concentré obtenu par ultrafiltration est ensuite traité par atomisation.

Le complexe est finalement obtenu sous forme d'une poudre beige clair, titrant au moins à 2 000 U.A./mg, compte non tenu des supports inertes, par exemple amidon surséché, éventuellement ajoutés avant ou après l'atomisation.

Le rendement global en activité protéolytique, qui est d'environ 70%, est supérieur à celui qui serait obtenu industriellement par précipitations successives ou passages sur colonne.

En résumé

Il convient, pour obtenir le complexe protéolytique désiré, à partir d'une souche de Streptomyces fradiae, de maintenir le pH du milieu de culture entre 7,5 et 7 par addition d'une solution d'un acide minéral ou organique, puis d'effectuer l'extraction du complexe protéolytique par filtration en présence d'un agent clarifiant puis ultrafiltration sur une membrane ayant un seuil de coupure correspondant à un poids moléculaire compris entre 2 000 et 12 000, et enfin atomisation.

Il est particulièrement adéquat d'utiliser comme souche de Streptomyces fradiae la souche typique WAKSMAN 3535 déposée à l'ATCC sous le n° 10745, de maintenir le pH du milieu de culture entre 7,5 et 7 au moyen d'acide chlorhydrique 3N, et d'employer comme membrane d'ultrafiltration une membrane à base de polysulfone vendue par la Société MILLIPORE sous la dénomination PTGC.

Caractéristiques du nouveau complexe

. présence (déterminée par enzymoélectrophorèse) des protéases du type II, III, IV, à l'exclusion des protéases du type $I_a$, $I_b$ et des peptidases;
. prépondérance de la protéase du type II qui représente au moins 40% de l'activité protéolytique totale, les protéases du type III ou IV représentant au plus 30% de cette activité totale chacune

Caractéristiques des protéases constitutives de ce nouveau complexe

. Protéase du type II. Poids moléculaire voisin de 18 000; point isoélectrique voisin de 9,0; par électrophorèse à pH 8,0, déplacement vers la cathode à vitesse relativement faible.
. Protéase du type III. Poids moléculaire voisin de 14 000; point isoélectrique voisin de 9,0; par électrophorèse à pH 8,0, déplacement vers la cathode à vitesse moyenne;
. Protéase du type IV . Poids moléculaire voisin de 16 500; point isoélectrique voisin de 9,0; par électrophorèse à pH 8,0, déplacement vers la cathode à vitesse relativement forte.

APPLICATIONS EN ZOOTECHNIE

Les applications en zootechnie du complexe selon l'invention conduisent aux résultats les meilleurs lorsqu'elles sont effectuées conformément aux conditions suivantes:

Utilisation d'aliments ayant une teneur élevée en protéines brutes

On a constaté que le complexe stimulait la croissance des animaux en tendant à privilégier la protéinogenèse par rapport à la lipogenèse. Par exemple, on a observé que les carcasses des veaux et des porcs traités étaient parfois mieux classées parce qu'elles étaient plus musclées et moins grasses, mais que ces améliorations étaient irrégulières.

On a alors supposé que cette tendance du complexe à stimuler l'anabolisme protéique ne pouvait s'exprimer pleinement que si l'aliment utilisé avait une teneur suffisante en protéines brutes. L'expérience a effectivement confirmé que, pour obtenir avec régularité une stimulation notable de l'anabolisme protéique, une des conditions à observer était d'utiliser un aliment relativement riche en protéines brutes, c'est-à-dire ayant un taux élevé de Matière Azotée Totale (en abrégé: M.A.T.).

Par exemple, ce taux de M.A.T. doit être:

≥ 23% pour les aliments destinés au démarrage des poulets,

≥ 22% pour les aliments d'allaitement destinés aux veaux ou aux agneaux;

≥ 20% pour les aliments de sevrage destinés aux porcelets;

≥ 16% pour l'aliment unique destiné aux truies en gestation et en lactation.

L'utilisation de tels aliments ne correspond pas, en général, à la pratique courante parce que:

- les animaux ne sont pas toujours capables d'assimiler complètement un aliment ayant un taux de M.A.T. élevé; les protéines non digérées peuvent alors provoquer un déséquilibre de la flore intestinale par développement excessif des bactéries protéolytiques, et une augmentation de la fréquence des diarrhées. L'utilisation du complexe permet précisément d'éliminer ces risques parce qu'elle confère aux animaux l'aptitude à assimiler efficacement les aliments ayant un taux de M.A.T. élevé;

- les aliments ayant un taux de M.A.T. élevé ont un prix supérieur à celui des aliments ordinaires et leur supplémentation en complexe augmente encore ce prix. Toutefois, cette augmentation peut être compensée par le fait que le complexe permet d'utiliser des aliments ayant une teneur en énergie inférieure aux normes courantes. De toute façon, en raison de l'amélioration importante des performances zootechniques obtenues, le bilan économique global de l'opération demeure très avantageux.

## Utilisation d'aliments destinés aux mammifères en période de gestation, de lactation et de sevrage

On a observé que le complexe particulier, décrit dans la présente demande de brevet, stimule l'activité exocrine du pancréas, ce qui apparaît nouveau eu égard au brevet français n° 2 034 599. Cette stimulation se traduit notamment, au cours d'expériences sur le rat, par une augmentation importante de la concentration intrapancréatique de toutes les enzymes qui seront excrétées par le canal pancréatique; cette augmentation peut atteindre 50% pour l'amylase, 70% pour le trypsinogène et le chymotrypsinogène, 80% pour la lipase. C'est pourquoi le complexe particulier, décrit par la présente demande de brevet, doit être considéré comme une substance nouvelle, douée de propriétés nouvelles et inattendues.

En raison de cette stimulation de l'activité exocrine du pancréas, on a supposé que le complexe était particulièrement efficace lorsque le pancréas risquait d'être déficient (cas des femelles en fin de gestation), et lorsqu'il devait fournir un travail exceptionnel de production (cas des femelles allaitant une portée nombreuse) ou d'adaptation (cas des jeunes mammifères en période de sevrage). Les exemples suivants sont en accord avec cette hypothèse de travail:

a) Le pancréas des femelles en fin de gestation est souvent déficient parce que le développement important du volume de l'utérus comprime tous les organes digestifs. L'addition du complexe dans l'aliment distribué à ces femelles permet alors de rétablir un bon fonctionnement du pancréas, et par suite d'obtenir une augmentation du poids des portées à la naissance.

Par exemple, avec un aliment unique pour truies gestantes ou allaitantes ayant un taux de M.A.T. de 16%, l'addition du complexe à la dose de 120 U.A. par g d'aliment a permis d'obtenir une augmentation de 17% pour le poids des portées à la naissance: 15 kg au lieu de 12,8 kg (10,7 porcelets de 1,4 kg au lieu de 10,2 porcelets de 1,25 kg).

b) La consommation alimentaire quotidienne des femelles allaitant une portée nombreuse doit augmenter rapidement: entre la fin de la gestation et le huitième jour de la lactation, elle doit être multipliée par 2,5 (environ 45 g au lieu de 18 g pour les rates, 350 g au lieu de 140 g pour les lapines, 6 kg au lieu de 2,4 kg pour les truies).

Le pancréas de ces femelles en lactation doit donc fournir un travail supplémentaire considérable, et c'est précisément dans ces conditions que l'action du complexe se montre la plus nette.

Par exemple, avec un aliment unique pour truies gestantes ou allaitantes ayant un taux de M.A.T. de 17%, l'addition du complexe à la dose de 120 U.A. par g d'aliment a permis d'obtenir une augmentation de 25% pour le poids des portées au sevrage à 21 jours: 54 kg au lieu de 43 kg. (9,4 porcelets de 5,7 kg au lieu de 9,1 porcelets de 4,7 kg).

Dans un autre essai effectué dans des conditions voisines, mais avec sevrage à 28 jours au lieu de 21 jours, l'augmentation du poids des portées a été également de l'ordre de 25%: 52 kg au lieu de 41 kg à 21 jours (9,7 porcelets de 5,4 kg au lieu de 8,5 porcelets de 4,8 kg) et 69 kg au lieu de 55 kg au sevrage à 28 jours (9,7 porcelets de 7,1 kg au lieu de 8,5 porcelets de 6,5 kg).

Cette augmentation du poids des portées va de pair avec une diminution de la consommation alimentaire nécessaire au maintien des truies en bon état, diminution qui, sur l'ensemble d'une année, est de l'ordre de 10% (environ 1 000 kg au lieu de 1 100 kg).

c) En période de sevrage, les jeunes mammifères doivent passer de la consommation du lait maternel à celle des aliments composés, et leur pancréas doit s'adapter rapidement à ce changement important. L'addition du complexe dans ces aliments composés permet alors de faciliter cette adaptation. Par exemple, avec un aliment de sevrage pour porcelets entre 5 et 10 kg, ayant un taux de M.A.T. de 23%, l'addition du complexe à la dose de 120 U.A. par g d'aliments a permis d'obtenir une augmentation de 15% du Gain de Poids Moyen Quotidien (216 g au lieu de 188 g) et une diminution de 15% à l'Indice de Consommation (1,59 au lieu de 1,83). Plus encore que dans le cas des porcelets, l'action du complexe au moment du sevrage peut être importante dans le cas des lapereaux et des jeunes ruminants (chevreaux, agneaux, veaux). Pour ces animaux, l'augmentation du Gain de Poids Moyen Quotidien sous l'action du complexe peut atteindre 40% (47 g au lieu de 33 g pendant la semaine suivant le sevrage des lapereaux de 5 semaines, 797 g au lieu de 573 g pendant le mois suivant le sevrage des veaux de 2 mois).

### Définition des doses extrêmes utilisables

On a constaté que, pour donner avec régularité des améliorations importantes, le complexe doit être utilisé à une dose se situant dans un intervalle relativement étroit, c'est-à-dire à une dose comprise entre 40 et 160 U.A. par g d'aliments.

Dans le cas de l'alimentation des truies allaitantes, la dose utilisée est de 120 U.A. par g d'aliments et l'augmentation du poids des portées de porcelets au sevrage à 21 jours est de 25%. Si cette dose est fixée à 200 U.A./g, l'augmentation de ce poids n'est plus que de 4%: 49 kg au lieu de 47 kg, soit 8,2 porcelets de 6,0 kg au lieu de 8,4 porcelets de 5,6 kg.

Dans ce cas de l'alimentation des porcelets, la dose utilisée est de 120 U.A./g et l'amélioration des performances zootechniques est de 15%. Si cette dose est fixée à 200 U.A./g, l'amélioration des performances devient irrégulière; au mieux, elle reste de 15% après 3 semaines d'essai, mais devient nulle après 5 semaines d'essai.

**Revendications**

1. Un procédé de production d'un complexe protéolytique formé par fermentation d'une souche de Streptomyces fradiae, caractérisé en ce que le pH du milieu de culture est maintenu entre 7,5 et 7,0 par addition d'une solution d'un acide minéral ou organique et en ce que l'extraction du complexe protéolytique est effectuée par filtration en présence d'un agent clarifiant, puis ultrafiltration sur une membrane ayant un seuil de coupure correspondant à un poids moléculaire compris entre 2.000 et 12.000 et enfin, atomisation.

2. Un procédé selon la revendication 1, dans lequel la souche de Streptomyces fradiae est la souche WAKSMAN 3535, déposée à l'ATCC sous le n° 10.745.

3. Un procédé selon la revendication 1, dans lequel le pH du milieu de culture est ajusté par addition d'acide chlorhydrique 3N.

4. Un procédé selon la revendication 1, dans lequel la membrane d'ultrafiltration est celle à base de polysulfone vendue par la Société MILLIPORE sous la dénomination PTGC.

5. Le complexe protéolytique obtenu à partir de Streptomyces fradiae par le procédé de la revendication 1.

6. Le complexe protéolytique selon la revendication 5, dans lequel le titre est moins égal à 2.000 Unités Anson/mg.

7. Le complexe protéolytique selon la revendication 5 ou la revendication 6, composé des protéases du type II, III, IV à l'exclusion des protéases du type $I_a$, $I_b$ et des peptidases, dans lequel la protéase du type II représente au moins 40% de l'activité protéolytique totale et dans lequel les protéases du type III ou IV représentent au plus 30% de l'activité protéolytique totale, chacune

8. Application du complexe protéolytique selon l'une des revendications 5 à 7, à la supplémentation des aliments destinés aux animaux.

9. Aliments composés destinés aux animaux, présentant une quantité du complexe protéolytique selon l'une des revendications 5 à 7, correspondant à une activité protéolytique comprise entre 40 et 160 Unités Anson par g d'aliments.

10. Aliments composés destinés au sevrage des jeunes mammifères, présentant une quantité du complexe protéolytique selon l'une des revendications 5 à 7 correspondant à une activité protéolytique comprise entre 40 et 160 Unité Anson par g d'aliments.

11. Aliments composés destinés aux femelles en gestation ou en lactation, présentant une quantité du complexe protéolytique selon l'une des revendications 5 à 7 correspondant à une activité protéolytique comprise entre 40 et 160 Unités Anson par g d'aliments.

12. Aliments composés destinés aux lapines ou aux truies en gestation ou en lactation, présentant un taux de Matière Azotée Totale au moins égal à 16%, et une quantité du complexe protéolytique selon l'une des revendications 5 à 7 correspondant à une activité protéolytique comprise entre 40 et 160 Unités Anson par g d'aliments.

13. Aliments composés destinés aux porcelets en cours de sevrage ou de post-sevrage présentant un taux de Matière Azotée Totale au moins égal à 20% et une quantité du complexe protéolytique selon l'une des revendications 5 à 7 correspondant à une activité protéolytique comprise entre 40 et 160 Unités Anson par g d'aliments.

## Claims

1. A process for producing a proteolytic complex obtained through the fermentation of a strain of Streptomyces fradiae, wherein the pH value of the broth is kept between 7,5 and 7,0 by adding a solution of an inorganic or organic acid, and wherein the extraction of the proteolytic complex is performed using filtration in the presence of a clarifying agent, then ultrafiltration on a membran having a cutting threshold corresponding to a molecular weight ranging between 2,000 and 12,000, and finally atomization.

2. A process according to claim 1 wherein the strain of Streptomyces fradiae is the WAKSMAN's strain 3535 deposited at ATCC under the admission number 10.745.

3. A process according to claim 1 wherein the pH value of the fermentation broth is adjusted by adding 3N solution of hydrochloric acid.

4. A process according to claim 1 wherein the membrane used for ultrafiltration is that based on polysulphone sold by the Company MILLIPORE under the Trade Name PTGC.

5. The proteolytic complex obtained from Streptomyces fradiae by the process of claim 1.

6. The proteolytic complex according to claim 5 wherein the proteolytic titer is at least 2.000 U Anson/mg.

7. The proteolytic complex according to claim 5 or claim 6 consisting of proteases of type II, III, IV excluding the proteases of type $I_a$, $I_b$ and the peptidases, wherein protease of type II represents at least 40 % of the whole proteolytic activity and wherein proteases of type III or IV represent each at the highest 30 % of the whole proteolytic activity.

8. Use of the proteolytic complex according to any of claims 5 to 7 for the supplementation of the food intended for the animals.

9. Mixed chows intended for the animals, containing such an amount of the proteolytic complex according to any of claims 5 to 7, which corresponds to a proteolytic activity comprised between 40 and 160 Units Anson per g. of chow.

10. Mixed chow intended to the suckling of young mammals which contain such an amount of the proteolytic complex according to any of claims 5 to 7 which corresponds to a proteolytic activity ranging between 40 and 160 Units Anson per g. of chow.

11. Mixed chow intended to pregnant or lactating females, containing such an amount of the proteolytic complex according to any of claims 5 to 7 which correspond to a proteolytic activity ranging from 40 and 160 Units Anson per g. of chow.

12. Mixed food intended to femal rabbits or sows during the gestation or lactation period, having a content in whole nitrogenous material at least equal to 16 % and such an amount of the proteolytic complex according to claims 5 to 7 which corresponds to a proteolytic activity ranging from 40 and 160 Units Anson per g. of food.

13. Mixed food intended to piglets during withdrawal or post-withdrawal period having a content in whole nitrogenous material at least equal to 20 % and such an amount of the proteolytic complex according to claims 5 to 7 which corresponds to a proteolytic activity ranging between 40 and 160 Units Anson per g. of food.

**Patentansprüche**

1. Verfahren zur Herstellung eines proteolytischen durch Gärung eines Stamm von Streptomyces fradiae gewonnenen Komplexes dadurch gekennzeichnet, dass der pH wert des Zuchtmediums zwischen 7,5 und 7,0 mit Hilfe der Zugabe einer Lösung einer anorganischen oder organischen Säure, liegt und dass die Extraktion des proteolytischen Komplexes durch Filtrieren in Gegenwart eines Klärmittel, durchgeführt wird, danach auf einen der ein Abschnittanbruch einem Molekulargewicht zwischen 2.000 und 12.000 aufweisende, entsprechende Membrane ultrafiltriert und schliesslich man sprüh trocknet.

2. Ein Verfahren nach Anspruch 1 worin die Stamm von Streptomyces fradiae die Waksman's Stamm 3535 ist, die bei ATCC unter der Number 10.745 vorgelegt wurde.

3. Ein Verfahren nach Anspruch 1, worin der pH Wert des Zuchtmediums mittels Zugabe 3N Chlorwasserstoff, eingestellt wird.

4. Ein Verfahren nach Anspruch 1 worin die Ultrafiltration membran, die auf Basis von Polysulfone ist, die durch die Firma MILLIPORE unter des Handelsname PTGC verkauft ist, ist.

5. Der von Streptomyces fradiae nach dem Verfahren des Anspruches 1 hergestellte proteolytische Komplex.

6. Der proteolytische Komplex nach Anspruch 5 worin das Gehalt mindenstens gleich wie 2.000 Anson Einheiten/mg, ist.

7. Der proteolytische Komplex nach Anspruch 5 oder Anspruch 6 der aus Proteasen des Types II, III, u. IV, mit der Ausschuss von Proteasen von Typ $I_a$ und $I_b$, und von Peptidasen besteht,in welchem die Protease von Typ II, mindenstens 40 % der gesamt proteolytischen Aktivität darstellt und in welchem die Proteasen von Typ III und IV jeweilig höchstens 30 % der gesamt proteolytischen Aktivität, darstellen.

8. Verwendung des proteolytischen Komplexes nach einer der Ansprüche 5 bis 7, fur die Ergänzung der fur die Tiere bestimmten Futterungsmitteln.

9. Die für die Tiere bestimmte Futterungsmittel, die eine Menge an proteolytische Komplex nach einer der Ansprüche 5 bis 7, der eine proteolytische Wirkung zwischen 40 und 160 Anson Einheiten per g. Nahrungsmittels entspricht, enthalten.

10. Die für das Absetzen der jungen Säugetieren bestimmte Futterungsmittel, die eine solche Menge an proteolytischen Komplex nach einer der Ansprüche 5 bis 7 enthalten, die einer proteolytischen Wirkung zwischen 40 und 160 Anson Einheiten entspricht.

11. Die für die Schwangerschaft oder die Milchabsonderung bestimmte zusammengesetzte Futterungsmittel, die eine solche Menge an proteolytische Komplex nach einer der Ansprüche 5 bis 7 vorstellen, die einer proteolytischen Wirkung zwischen 40 und 160 Anson Einheiten entspricht.

12. Die für die Häsinnen oder Sanen, während Schwangerschaft oder Milchabsonderung zusammengesetzte Futterungsmittel, die ein Gehalt an gesamt Stickstoffhaltige Material mindestens gleich wie 16 % aufweisen und die, eine solche Menge an proteolytischen Komplex nach einer der Ansprüche 5 bis 7 enthalten, die einer proteolytischen Wirkung zwischen 40 und 160 Anson Einheiten per g. Futterung entspricht.

13. Die für die Ferkeln im Absetzen oder Nachabsetzen Futterungsmittel, die ein Gehalt an gesamt Stickstoffhaltige Material mindestens gleich wie 20 % aufweisen und die eine solche Menge an proteolytischen Komplex nach einer der Ansprüche 5 bis 7 enthalten, die einer proteolytischen Wirkung zwischen 40 and 160 Anson Einheiten per g. Futterung entspricht.